# EUROPEAN PATENT SPECIFICATION

(11) **EP 2 455 046 B1**
(45) Date of publication and mention of the grant of the patent: **26.02.2014**
(21) Application number: 10800010.0
(22) Date of filing: 13.07.2010
(51) Int. Cl.: A61H 3/06

(54) **ROAD GUIDE APPARATUS FOR A VISUALLY IMPAIRED PERSON**
STRASSENFÜHRUNGSVORRICHTUNG FÜR EINE PERSON MIT EINGESCHRÄNKTER SEHFÄHIGKEIT
APPAREIL DE GUIDAGE SUR LA ROUTE POUR UNE PERSONNE AVEUGLE

(30) Priority: 13.07.2009 KR 20090063517
(43) Date of publication of application: 23.05.2012
(73) Proprietor: Kim, Ji Hoon, Daegu 711-851 (KR)
(72) Inventor: Kim, Ji Hoon, Daegu 711-851 (KR)
(74) Representative: Mittler, Enrico
(86) International application number: PCT/KR2010/004534
(87) International publication number: WO 2011/007999

(56) References cited:
- JP-A- 6 343 669
- JP-A- 2000 084 018
- JP-A- 2004 309 305
- KR-A- 20070 114 637
- KR-A- 20090 020 857
- MORI H ET AL: "A robotic travel aid HITOMI", INTELLIGENT ROBOTS AND SYSTEMS '94. 'ADVANCED ROBOTIC SYSTEMS AND THE REAL WORLD', IROS '94. PROCEEDINGS OF THE IEEE/RSJ/GI INTERNATIONAL CO NFERENCE ON MUNICH, GERMANY 12-16 SEPT. 1994, NEW YORK, NY, USA,IEEE, vol. 3, 12 September 1994 (1994-09-12), pages 1716-1723, XP010142071, DOI: 10.1109/IROS.1994.407626 ISBN: 978-0-7803-1933-2
- T. YOSHIDA ET AL: "Braille block detection for autonomous mobile robot navigation", PROCEEDINGS. 2000 IEEE/RSJ INTERNATIONAL CONFERENCE ON INTELLIGENT ROBOTS AND SYSTEMS (IROS 2000) (CAT. NO.00CH37113), vol. 1, 1 January 2000 (2000-01-01), pages 633-638, XP55043707, DOI: 10.1109/IROS.2000.894675 ISBN: 978-0-78-036348-9

## Description

### [Technical Field]

The present invention relates to a road guide apparatus for a visually impaired person, and more particularly, to a road guide apparatus for a visually impaired person which not only senses the yellow color of a Braille block installed on a road to guide the visually impaired person, but also reads information from an RFID tag to guide the visually impaired person.

### [Background Art]

A visually impaired person walks mostly with a stick or with the support of a guide dog, and is guided with Braille blocks installed on a road when he/she walks.

A guide dog may not be trusted 100% for any unexpected circumstances even if the dog is well trained. Also, even if the Braille blocks are installed, the visually impaired person may feel difficult and walk slow following the Braille blocks.

If the visually impaired person approaches a roadway by mistake, accident may occur due to a passing vehicle.

A guard rail should be installed between a sidewalk and a road to prevent the visually impaired person from approaching the road, but there are many issues in installing the guard rail in cities and many difficulties are associated with the guard rail, and thus the guard rail is not installed usually.

Also, there may be trash bins or other installations in the boundary between the roadway and the road, the visually impaired person may feel difficult while walking.

Accordingly, it is essential to inform the visually impaired person of the information on such installations.

To help the visually impaired person walk without difficulty, a stick with a built-in RFID reader has been developed to read an RFID tag installed within Braille blocks and guide the condition and information of a road.

However, the RFID tag is slow in response time and thus the visually impaired person should frequently stop walking, and the existing Braille blocks are not utilized well. The Braille blocks are the most fundamental means for guiding the visually impaired person and may guide the visually impaired person when the RFID tag or receiver has a problem. However, it is difficult for the visually impaired person to walk only with the sense of the sole of the foot. That is, if the visually impaired person wears shoes with a thick sole, he/she may not sense the Braille of the Braille blocks.

Also, as vehicles should pass by a pedestrian crossing, it is difficult to install the Braille blocks on the pedestrian crossing.

MORI H ET AL: "A robotic travel aid HITOMI", INTELLIGENT ROBOTS AND SYSTEMS '94, 'ADVANCED ROBOTIC SYSTEMS AND THE REAL WORLD' IROS '94, PROCEEDINGS OF THE IEE/RSJ/GI INTERNATIONAL CONFERENCE ON MUNICH, GERMANY 12-16 SEPT, 1994, NEW YORK, NY, USA, IEEE, vol. 3, 12 September 1994, pages 1716-1723 disclosed a road guide apparatus as recited in the preamble of claim 1.

T. YOSHIDA ET AL: "Braille block detection for autonomous mobile robot navigation", PROCEEDINGS, 2000 IEEE/RSJ INTERNATIONAL CONFERENCE ON INTELLIGENT ROBOTS AND SYSTEMS (IROS 2000), vol. 1, 1 January 2000, pages 633-638 discloses a method for the detection of Braille blocks, which are originally used for visually handicapped people, for autonomous robot navigation. Yellow color is used for detecting Braille blocks.

JP-2004 309305 A discloses the use of RFID tags to provide the user with additional information. Particularly, it is a standard orpion to include RFID tags in Braille blocks.

### [Disclosure]

### [Technical Problem]

The present invention has been made to solve the problems and it is an object of the present invention to provide a road guide apparatus for a visually impaired person which not only senses an RFID tag but also the yellow color of a Braille block and guides the visually impaired person.

It is another object of the present invention to provide a road guide apparatus in which a yellow color guide line is formed in a pedestrian crossing where a Braille block cannot be installed, to thereby guide the visually impaired person without hindering the driving of vehicles.

### [Technical Solution]

In order to achieve the object of the present invention, a road guide apparatus for a visually impaired person which comprises the features recited in claim 1 is provided.

### [Advantageous Effect]

As described above, a road guide apparatus for a visually impaired person according to the present invention not only enables a visually impaired person to read information stored in an RFID tag with a road detector but also senses the yellow color of the Braille blocks to guide the road for the visually impaired person, and helps the visually impaired person easily find the road without sensing the Braille of the Braille blocks and guides the visually impaired person at a fast speed even if the RFID tag reading speed is slow.

Also, if the yellow color alone is installed in a pedestrian crossing or other places where the Braille blocks are hard to install, a visually impaired person may be easily guided.

### [Brief Description of Drawings]

FIG. 1 is a perspective view of a road guide apparatus for a visually impaired person according to the present invention.
FIG. 2 is a plan view of the road guide apparatus for the visually impaired person according to the present invention.
FIG. 3 is a lateral view of the road guide apparatus road guide apparatus for the visually impaired person according to the present invention.
FIG. 4 illustrates a pedestrian crossing where the road guide apparatus for a visually impaired person according to the present invention is used.
FIG. 5 illustrates a control unit which is used for the road guide apparatus for the visually impaired person according to the present invention.
FIG. 6 illustrates a yellow color detecting sensor which is used for the road guide apparatus for the visually impaired person according to the present invention.

### [Best Mode]

Below, exemplary embodiments will be described in detail with reference to accompanying drawings so as to be easily realized by a person having ordinary knowledge in the art. The exemplary embodiments may be embodied in various forms without being limited to the exemplary embodiments set forth herein. Descriptions of well-known parts are omitted for clarity, and like reference numerals refer to like elements throughout.

A road guide apparatus for a visually impaired person which includes a handle and wheels attached to a main body detects the yellow color as the typical color of a Braille block 2 and guides a visually impaired person even if the visually impaired person may not sense the Braille block 2 by himself/herself, and reads detailed information from an RFID tag in a place where direction is switched. In a road sensing apparatus 10, a handle rod 20 is fixed in one side of a main body 30 via a hinge 22, and a control unit 50 is installed within the main body 30, and a front detecting sensor 41, a left detecting sensor 42 and a right detecting sensor 43 of a yellow color detecting unit 40 are installed in the front and both sides of the main body 30, respectively. A yellow color guide line 70 is painted in a pedestrian crossing 1 so as to be recognized by the yellow color sensing unit 40 and guide the visually impaired person.

In the road sensing apparatus 10, the handle rod 20 is rotatably fixed at one side of a rear end of the main body 30 via the hinge 22, and a handle 21 is formed in an upper end of the handle rod 20 to be easily grabbed by the visually impaired person.

The control unit 50 is formed within the main body 30, and is connected to the yellow color detecting unit 40 and to a tag detecting sensor 60. The yellow color detecting sensor 40 is connected to the control unit 50 and senses the yellow color of the Braille block 2 and transmits a first signal to the control unit 50. The tag detecting sensor 60 is connected to the control unit 50 and senses a signal of an RFID tag installed on a road and transmits a second signal to the control unit 50.

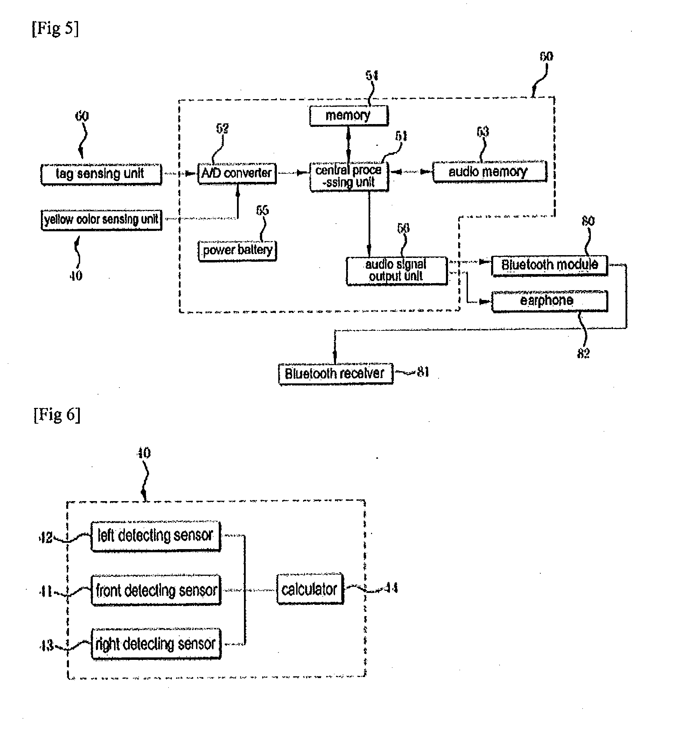

As shown in FIG. 5, the control unit 50 has a central processing unit 51 for processing the first and second signals, and an audio signal output unit 56 for transmitting information to a Bluetooth module 80 or an earphone 82 so as to be recognized by the visually impaired person. The central processing unit 51 is connected to a memory 54 and an audio memory 53 and receives the content of the RFID tag sensed by the tag sensing unit 60 through an A/D converter 52, which also receives the first signal transmitted by the yellow color detecting unit 40.

The control unit 50 is connected to the central processing unit 51 and the memory 54 and calculates alarm information within the memory 54 to transmit the information to the audio signal output unit 56. The tag sensing unit 60 which senses a signal of the RFID tag and the yellow color sensing unit 40 which senses the yellow color are connected to the central processing unit 51 via the A/D converter 52. A power battery 55 is built in the control unit 50.

The audio memory 53 stores therein an audio signal and transmits the audio signal to the audio signal output unit 56 according to a signal of the central processing unit 51. The audio signal output unit 56 is connected to the Bluetooth module 80 and performs a short distance wireless communication with the Bluetooth receiver 81 which is put on the ear of the visually impaired person, to notify information of a road.

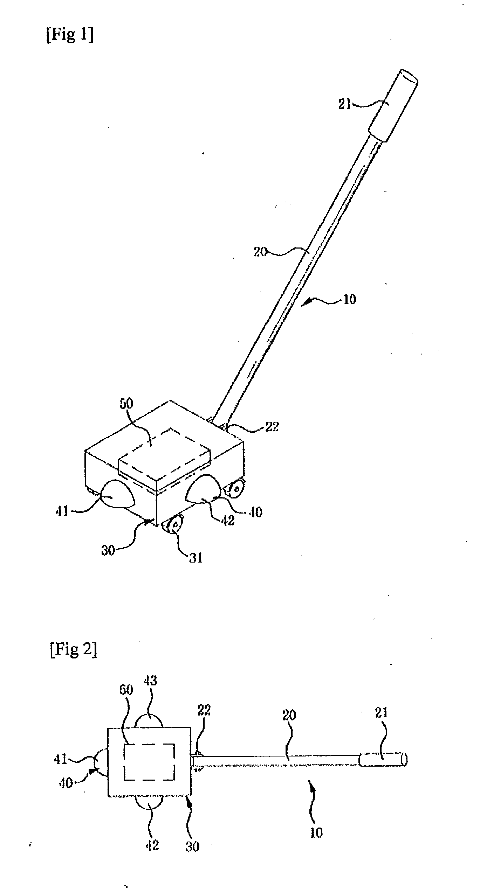

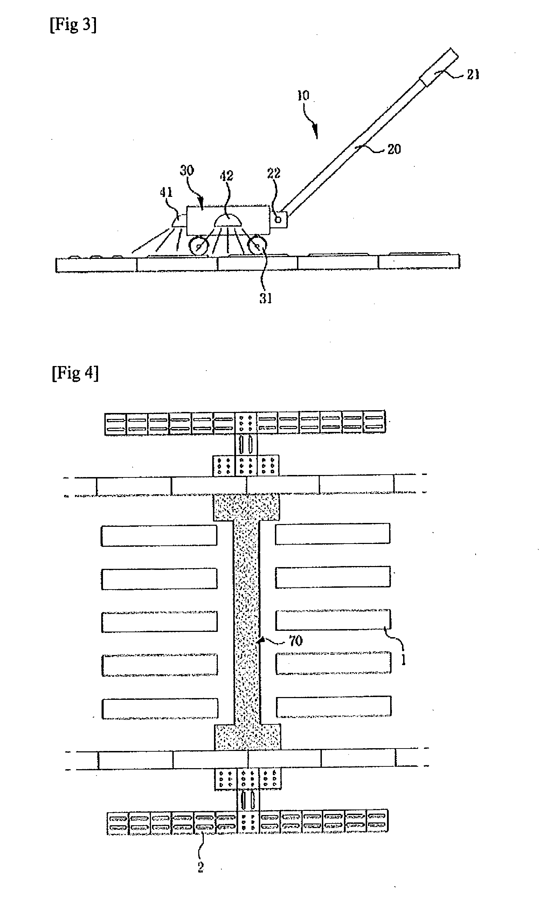

The yellow color sensing unit 40 is installed with a color detecting sensor to sense a yellow line in a width wider than the width of a given Braille block 2. As shown in FIGS. 1 to 4, the yellow color sensing unit 40 is provided with the front detecting sensor 41, the left detecting sensor 42 and the right detecting sensor 43 in the front and left and right sides of the main body 30, respectively.

The front detecting sensor 41 and the left and right detecting sensors 42 and 43 are connected to a calculator 44, which combines the output signal, of the sensors 41, 42, 43 into the first signal of the yellow color detecting unit 40 and is connected to the central processing unit 51 via the A/D converter 52.

Once located within the width of the Braille block 2, the front detecting sensor 41, the left detecting sensor 42 and the right detecting sensor 43 sense the yellow color and guide the visually impaired person to go straight.

If one of the left and right detecting sensors 42 and 43 does not sense the yellow color, the visually impaired person is guided to move in an opposite direction of the sensor that did not sense the yellow color. If the three sensors sense the yellow color again, the visually impaired person is guided to go straight.

If the front detecting sensor 41 does not sense the yellow color while the visually impaired person goes straight, he/she is guided to move to the left or right.

Accordingly, as all of the three sensors do not sense the yellow color in the yellow line which is narrower in width than the Braille block 2, they do not operate.

To ensure sensing of the yellow color sensing unit 40, the yellow color guide line 70 is painted in the same width of the Braille block 2 in the center of the pedestrian crossing 1, and the yellow color guide line 70 is painted to be connected to the Braille block 2 on the road.

An RFID tag is fixed to the directional switch area and the caution area of the Braille block 2 installed in the starting and ending points of the pedestrian crossing 1, and the road sensing apparatus 10 reads the tag and guides the starting of the pedestrian crossing 1 with an audio.

## Claims

1. A road guide apparatus for a visually impaired person which comprises a road sensing apparatus comprising a main body of the road sensing apparatus, wheels attached to the main body, a handle rod fixed to the main body via a hinge and a handle formed in the upper end of the handle rod and a control unit installed within the main body, the control unit comprising a central processing unit for processing signals and an audio signal output unit for transmitting information to a Bluetooth module or to an earphone so as to be recognized by the visually impaired person, **characterized in that** the control unit is connected to a yellow color sensing unit and a tag sensing unit, and the yellow color sensing unit senses the yellow color of a Braille block or a yellow line and transmits a first signal to the control unit, and the tag sensing unit senses a signal of the RFID tag installed on the road and transmits a second signal to the control unit, wherein the yellow color sensing unit (40) is installed with a color detecting sensor to detect the yellow line in a width that is wider than the width of a given Braille block (2), the yellow color sensing unit (40) being provided with a front detecting sensor (41), a left detecting sensor (42) and a right detecting sensor (43) in the front and left and right sides of the main body, respectively, and the front detecting sensor (41) and the left and right detecting sensors (42, 43) are connected to a calculator (44), which combines the output signals of the detecting sensors (41, 42, 43) into the first signal of the yellow color detecting unit (40) and is connected to the central processing unit (51) through an A/D converter (52), so that in use, once located within the width of the Braille block (2), the front detecting sensor (41) and the left detecting sensor (42) and the right detecting sensor (43) sense the yellow color and guide the visually impaired person to go straight, whereas, if one of the left and right detecting sensors (42, 43) does not sense the yellow color, the visually impaired person is guided to move in an opposite direction of the sensor that did not sense the yellow color and, if the front detecting sensor (41) does not sense the yellow color while the visually impaired person goes straight, the visually impared person is guided to move to the left or right.

## Patentansprüche

1. Straßenführungsvorrichtung für eine sehbehinderte Person, mit einer Straßenerfassungsvorrichtung, die einen Hauptkörper der Straßenerfassungsvorrichtung, an dem Hauptkörper angebrachte Räder, eine an dem Hauptkörper über ein Gelenk angebrachte Handhabungsstange sowie einen an dem oberen Ende der Handhabungsstange ausgebildeten Handgriff aufweist, und mit einer in dem Hauptkörper installierten Steuereinheit, wobei die Steuereinheit eine zentrale Verarbeitungseinheit zum Verarbeiten von Signalen sowie eine Audiosignal-Ausgabeeinheit zum Übertragen von Information zu einem Bluetooth-Modul oder zu einem Kopfhörer aufweist, um von der sehbehinderten Person wahrgenommen zu werden,
**dadurch gekennzeichnet, dass** die Steuereinheit mit einer GelbErfassungseinheit zum Erfassen der Farbe Gelb und mit einer TagErfassungseinheit verbunden ist, und dass die Gelb-Erfassungseinheit die gelbe Farbe eines Braille-Blocks oder eine gelbe Linie erfasst und ein erstes Signal zu der Steuereinheit überträgt, und die TagErfassungseinheit ein Signal des auf der Straße installierten RFID-Tags erfasst und ein zweites Signal zu der Steuereinheit überträgt, wobei in der Gelb-Erfassungseinheit (40) ein Farbdetektionssensor installiert ist, um die gelbe Linie in einer größeren Breite als der Breite eines jeweiligen Braille-Blocks (2) zu detektieren, wobei die Gelb-Erfassungseinheit (40) mit einem vorderen Detektionssensor (41), einem linken Detektionssensor (42) und einem rechten Detektionssensor (43) an der Vorderseite, der linken Seite bzw. der rechten Seite des Hauptkörpers versehen ist, und wobei der vordere Detektionssensor (41) sowie der linke und der rechte Detektionssensor (42, 43) mit einem Rechner (44) verbunden sind, der die Ausgangssignale der Detektionssensoren (41, 42, 43) in das erste Signal der Gelb-Erfassungseinheit (40) kombiniert und mit der zentralen Verarbeitungseinheit (51) über einen A/D-Wandler (52) verbunden ist, so dass im Gebrauch, bei Anordnung innerhalb der Breite des Braille-Blocks (2), der vordere Detektionssensor (41) und der linke Detektionssensor (42) sowie der rechte Detektionssensor (43) die gelbe Farbe erfassen und die sehbehinderte Person zum geradeaus Gehen führen, während dann, wenn einer von dem linken und dem rechten Detektionssensor (42, 43) die gelbe Farbe nicht erfasst, die sehbehinderte Person zum Gehen in einer entgegengesetzten Richtung von dem Sensor, der die gelbe Farbe nicht erfasst hat, geführt wird, und dann, wenn der vordere Detektionssensor (41) die gelbe Farbe nicht erfasst, während die sehbehinderte Person geradeaus geht, die sehbehinderte Person zum Gehen nach links oder nach rechts geführt wird.

## Revendications

1. Appareil de guidage sur la route pour une personne malvoyante qui comprend un appareil détecteur de route comprenant un corps principal de l'appareil détecteur de route, des roues attachées au corps principal, une barre à poignée fixée au corps principal par une articulation et une poignée formée dans l'extrémité supérieure de la barre à poignée, et une unité de commande installée dans le corps principal, l'unité de commande comprenant une unité centrale pour traiter des signaux et une unité de sortie de signaux audio pour transmettre des informations à un module Bluetooth ou à un écouteur de manière à ce qu'elles soient reconnues par la personne malvoyante, **caractérisé en ce que** l'unité de commande est connectée à une unité détectrice de couleur jaune et à une unité détectrice d'étiquette, et l'unité détectrice de couleur jaune détecte la couleur jaune d'un bloc en Braille ou d'une ligne jaune et transmet un premier signal à l'unité de commande, et l'unité détectrice d'étiquette détecte un signal de l'étiquette RFID installée sur la route et transmet un second signal à l'unité de commande, dans lequel l'unité détectrice de couleur jaune (40) est installée avec un capteur détecteur de couleur pour détecter la ligne jaune dans une largeur qui est plus grande que la largeur d'un bloc en Braille donné (2), l'unité détectrice de couleur jaune (40) étant dotée d'un capteur de détection avant (41), d'un capteur de détection gauche (42) et d'un capteur de détection droit (43) à l'avant et sur les côtés gauche et droit du corps principal, respectivement, et le capteur de détection avant (41) et les capteurs de détection gauche et droit (42, 43) sont connectés à un calculateur (44) qui combine les signaux de sortie des capteurs de détection (41, 42, 43) en le premier signal de l'unité détectrice de couleur jaune (40) et est connecté à l'unité centrale (51) par le biais d'un convertisseur analogiquenumérique (52), de telle sorte que, pendant l'utilisation, une fois positionné dans la largeur du bloc en Braille (2), le capteur de détection avant (41), le capteur de détection gauche (42) et le capteur de détection droit (43) détectent la couleur jaune et guident la personne malvoyante pour qu'elle aille tout droit, alors que, si l'un des capteurs de détection gauche et droit (42, 43) ne détecte pas la couleur jaune, la personne malvoyante est guidée de sorte à se déplacer dans une direction opposée au capteur qui n'a pas détecté la couleur jaune et, si le capteur de détection avant (41) ne détecte pas la couleur jaune lorsque la personne malvoyante va tout droit, la personne malvoyante est guidée de manière à se déplacer vers la gauche ou la droite.
